# EUROPEAN PATENT SPECIFICATION

(11) **EP 4 215 127 B1**
(45) Date of publication and mention of the grant of the patent: **30.04.2025**
(21) Application number: 23152819.1
(22) Date of filing: 23.01.2023
(51) Int. Cl.: A61B 17/12

(54) **DELIVERY AND DETACHMENT SYSTEM FOR AN IMPLANTABLE INTRAVASCULAR TREATMENT DEVICE**
ABGABE- UND ABLÖSUNGSSYSTEM FÜR EINE IMPLANTIERBARE INTRAVASKULÄRE BEHANDLUNGSVORRICHTUNG
SYSTÈME DE POSE ET DE DÉTACHEMENT POUR DISPOSITIF DE TRAITEMENT INTRAVASCULAIRE IMPLANTABLE

(30) Priority: 24.01.2022 US 202217583091
(43) Date of publication of application: 26.07.2023
(73) Proprietor: DePuy Synthes Products, Inc., Raynham, MA 02767 (US)
(72) Inventor: BLUMENSTYK, David, Raynham, 02767 (US); SOLAUN, Daniel, Raynham, 02767 (US); LIEBOWITZ, Joshua, Raynham, 02767 (US)
(74) Representative: Carpmaels & Ransford LLP

(56) References cited:
- US-A1- 2012 041 470
- US-A1- 2022 117 606

## Description

### Field of the Invention

The present invention relates to a delivery and detachment system for an implantable intravascular treatment device used during an endovascular treatment or procedure. In particular example, the present invention is directed to a delivery and detachment system for implantation of an embolic coil in the intravascular treatment of a brain aneurysm.

### Description of Related Art

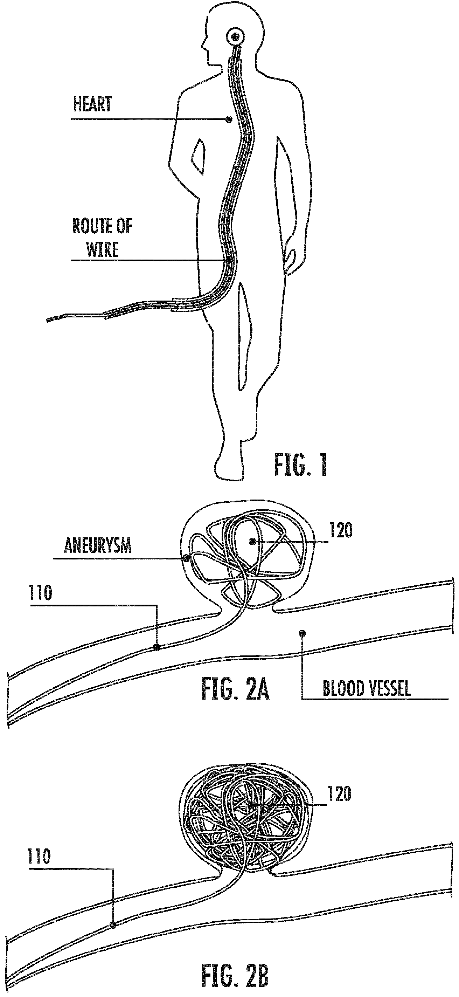

Implantable intravascular treatment devices are commonly used in the endovascular procedures or treatments of various vascular ailments, for example, brain aneurysms. A catheter is inserted into the femoral artery in patient's leg and guided by imaging navigated through the vessel to the target site in the brain where the aneurysm is located (Figure 1). With the distal end of the catheter properly positioned on a proximal side of the aneurysm, a microcatheter is tracked through the catheter to the proximal side of the aneurysm. A delivery and deployment system loaded with an implantable intravascular treatment device (e.g., embolic coil) is introduced via the microcatheter to the target site. During delivery to the target site, the implantable intravascular treatment device is secured within the delivery and deployment system via a securement wire. When properly positioned at the target site (e.g., at the location of the aneurysm) the implantable intravascular treatment device (e.g., embolic coil) is severed from the wire and thereby deposited within the aneurysm (Figure 2A). Severing of the embolic coil from the securement wire is typically achieved by passing of a small electrical current through the wire. This process is repeated until the area of the vessel with the weakened wall is tightly packed with numerous embolic coils occluding blow flow thereto thereby preventing rupture (Figure 2B).

US 2012/041470 A1 discloses a delivery and detachment system comprising an outer tube enclosing three bushings and a slider. The slider being slidably received in a first bushing, which is welded to the outer tube, and a second bushing, disposed proximally to the first bushing and is welded to the slider, being slidably received in the outer tube. Wherein the slider is temporarily fixed to the first bushing with a tack weld. The delivery and detachment system further comprising a cord received within a lumen of and secured to the slider, and configured to releasably secure an implant.

The present invention is directed to an improved system for reliable delivery of the implantable intravascular treatment device to a desired location in the vessel and ensuring consistent deployment when properly positioned at the target site.

### Summary of the Invention

The present invention is directed to a delivery and detachment system for an implantable intravascular treatment device. The system includes an outer delivery tube with a proximal end, an opposite distal end, and a lumen extending axially therethrough; the outer delivery tube having a radially inward stopping member. In addition, the system further includes a proximal inner tube telescopically receivable in the distal end and axially slidable in a proximal direction within the lumen of the outer delivery tube. The proximal inner tube has a proximal end, an opposite distal end, and a channel extending axially therethrough. Specifically, the proximal inner tube comprises a proximal section including the proximal end of the proximal inner tube and a distal section including the distal end of the proximal inner tube; wherein the proximal section and the distal section have different outer diameters forming a transition in outer diameter at an interface therebetween. In turn, the distal section of the proximal inner tube itself comprises two distal section components connected by an axially separable region; wherein the two distal section components comprise a first distal section component including the transition and a second distal section component arranged in a distal direction relative to the first distal section component. Still further the system includes a restricting element arranged between an outer surface of the second distal section component and an inner wall of the lumen of the outer delivery tube. When the axially separable region is axially separated: (i) the proximal section of the proximal inner tube is slidable in a proximal direction through the stopping member until the transition in the outer diameter of the first distal section component attached thereto engages the stopping member prohibiting axial movement in the proximal direction; and (ii) the restricting element maintaining in position the second distal section component within the outer delivery tube.

### Brief Description of the Drawing

The foregoing and other features of the present invention will be more readily apparent from the following detailed description and drawings illustrative of the invention wherein like reference numbers refer to similar elements throughout the several views and in which:
Figure 1 illustrates an exemplary use of for the present inventive delivery and detachment system loaded with an implantable intravascular treatment device being navigated through the femoral artery via a microcatheter to an intracranial aneurysm;
Figure 2A illustrates a microcatheter advanced to a proximal side of the aneurysm with a single embolic coil deposited therein;
Figure 2B illustrates the aneurysm of Figure 2A with multiple embolic coils packed therein following withdraw of the microcatheter;
Figure 3 is a longitudinal/axial cross-sectional view of an exemplary use of the present inventive delivery and detachment system loaded with an implantable intravascular treatment device tracked through the microcatheter once advanced through the guide catheter to the target site in the vessel;
Figure 4 is a detailed side view of components of an exemplary embodiment of the present inventive delivery and detachment system loaded with an implantable intravascular treatment device (e.g., embolic coil);
Figure 5 is a side view of distal section V of the present inventive delivery and detachment system loaded with an implantable intravascular treatment device (e.g., embolic coil) in Figure 4, wherein a substantial portion of the distal section V is cutaway to show the configuration of the inner components;
Figure 6 is a longitudinal cross-sectional view of proximal section VI of the present inventive delivery and detachment system in Figure 4;
Figure 7 is a longitudinal cross-sectional view of the proximal inner tube component alone comprising part of the present inventive delivery and detachment system of in Figure 4;

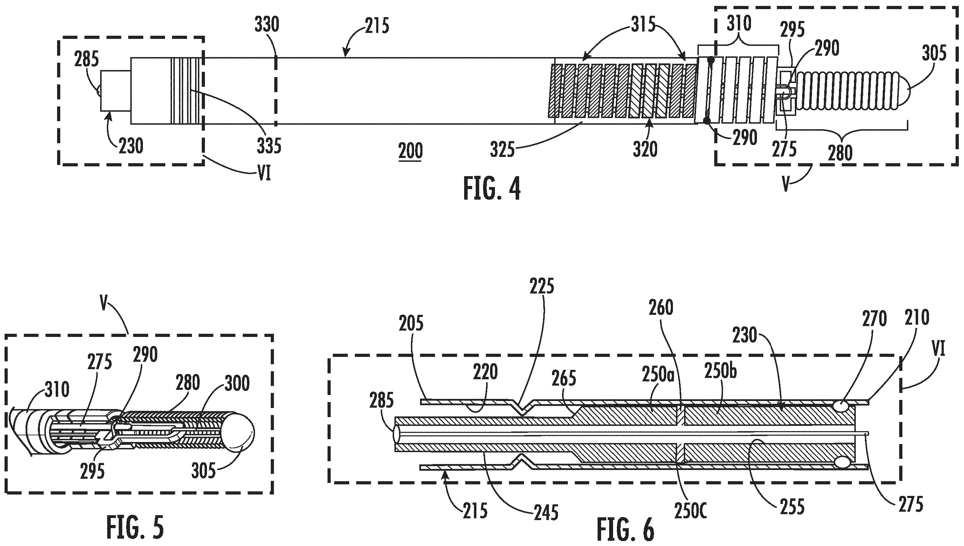

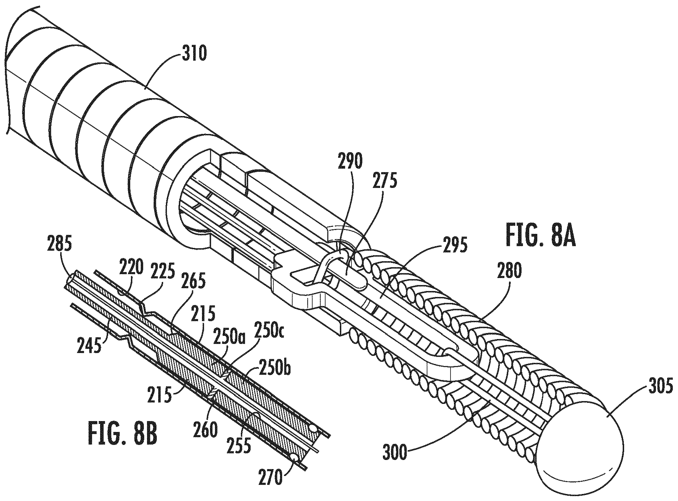

Figures 8A & 8B depict, while in a "natural state" (i.e., the state during which the interventionalist advances the implantable intravascular treatment device (e.g., embolic coil) through the vessel to the target site - prior to initiating movement (i.e., pulling) of the securement wire in a proximal direction (i.e., away from the patient) to release/deploy/detach the implantable intravascular treatment device); specifically, Figure 8A represents an isometric cutaway view of the distal section V of the delivery and detachment system loaded with an implantable intravascular treatment device (e.g., embolic coil) in Figure 4; while Figure 8B represents a corresponding longitudinal cross-sectional view of proximal section VI of the delivery and detachment system in Figure 4;

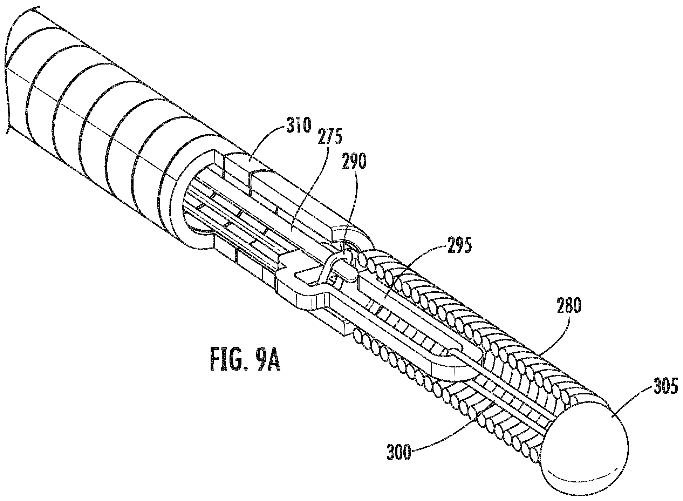

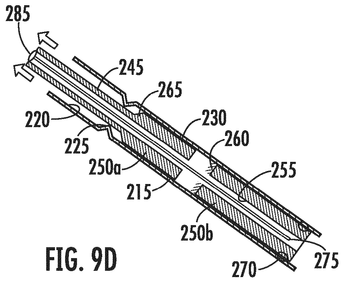

Figures 9A-9D depict various stages of a "deployment state" in which application of an axial force (i.e., pulling or sliding) in a proximal direction (i.e., away from the patient) on the proximal inner tube and securement wire attached thereto releases the implantable intravascular treatment device (e.g., embolic coil) from the distal end of the delivery and detachment system; specifically, Figures 9A-9C represent isometric cutaway views of the distal section V of the delivery and detachment system in Figure 4 at three sequential stages of movement in a proximal direction of the proximal inner tube resulting in release of the securement mechanism; during all three sequential stages of Figures 9A-9C, Figure 9D shows a corresponding longitudinal cross-sectional view of proximal section VI of the delivery and detachment system in Figure 4 illustrating the sliding in proximal direction (i.e., pulling) applied to the proximal inner tube axially separating (i.e., severing/rupturing/breaking) the two distal components along the struts/connectors (prior to the transition physically contacting the stopping member);
Figure 10A depicts, while in a "deposit state" wherein the implantable intravascular treatment device (e.g., embolic coil) is released from the delivery and detachment system, an isometric cutaway view of the distal section V of the delivery and detachment system loaded with an embolic coil in Figure 4;
Figure 10B depicts, while in a "deposit state" upon release of the implantable intravascular treatment device (e.g., embolic coil) pushing of the implantable intravascular treatment device (e.g., embolic coil) away from (i.e., clear of) the loop wire as a result of a small pre-load of the outer delivery tube during assembly of the delivery and detachment system;

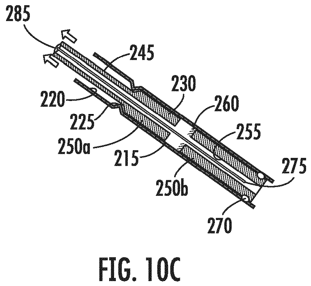

Figure 10C illustrates, while in a "deposit state," a longitudinal cross-sectional view of the proximal section VI of the delivery and detachment system in Figure 4 in a "halted stage" with the severed integral proximal unit of the inner tube (comprising the proximal section and severed first distal section component) self-contained within the outer delivery tube prevented from further axial movement in a proximal direction by the transition and stopping member directly physically engaging/contacting with one another while the severed second distal section component remains secured by the restricting element and self-contained within the outer delivery tube;
Figures 11A-11D are several exemplary strut designs or patterns representing the severable (weakened) section (prior to rupture) connecting first and second distal components comprising the distal section of the proximal inner tube of the present inventive delivery and detachment system; and
Figure 12 is a longitudinal cross-sectional view of an alternative configuration of the proximal inner tube of the present inventive delivery and detachment system, wherein an axially expandable and non-severable helical spring connects the two distal components comprising the distal section of the proximal inner tube of the present inventive delivery and detachment system.

### Detailed Description of the Invention

In the description, the terms "distal" or "proximal" are used in the following description with respect to a position or direction relative to the treating physician or medical interventionalist. "Distal" or "distally" are a position distant from or in a direction away from the physician or interventionalist. "Proximal" or "proximally" or "proximate" are a position near or in a direction toward the physician or medical interventionist. The terms "occlusion", "clot" or "blockage" are used interchangeably.

Figure 4 is a side view of the assembled present inventive delivery and deployment system 200 that includes an outer delivery tube (e.g., pusher) 215 open at both ends (e.g., proximal end 205 and opposite distal end 210) with a lumen 220 extending axially therethrough. A proximal inner tube 230 (e.g., proximal member) is telescopically slidable in an axial direction through the lumen 220 of the outer delivery tube 215, as illustrated in Figure 6 (representing an enlarged longitudinal cross-sectional view of proximal section VI of Figure 4). The maximum outer diameter of the proximal inner tube 230 is sized to be receivable and freely axially (longitudinally) slidable within the lumen 220 of the outer delivery tube 215. A stopping member 225 (i.e., crimping, projection or protrusion) (e.g., having a V-shape, hemispherical, square, rectangular or other cross-sectional geometric shape) extends radially inward from an inner wall of the outer delivery tube 215 thereby reducing the inner diameter of the lumen 220 at the stopping member.

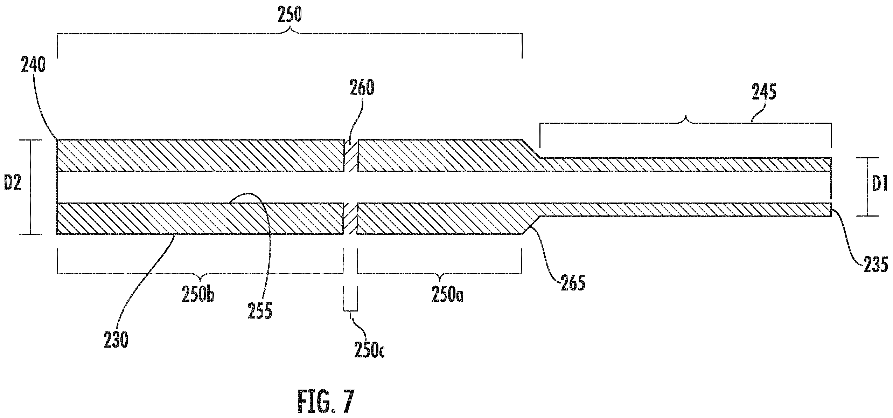

The proximal inner tube 230 alone is depicted in Figure 7 to include a proximal section 245 having a uniform first outer diameter (D1) and a distal section 250 having a uniform second outer diameter (D2), wherein D2 > D1 forming the transition 265 (i.e., shoulder or ground shelf) in outer diameter at the interface between the proximal and distal sections 245, 250, respectively. Proximal inner tube 230 has a uniform inner diameter from its proximal end 235 to its opposite distal end 240 defining a channel 255 therethrough. The wall thickness of the proximal section 245 is smaller (thinner in a radial direction) relative to the wall thickness of the distal section 250. The first outer diameter (D1) of the proximal section 245 is sized to slide freely in an axial direction through and beyond the stopping member 225 extending radially inward in the lumen 220 of the outer delivery tube 215, whereas a second outer diameter (D2) of the distal section 250 of the inner tube 230 is greater than the inner diameter of the stopping member 225. As a result of the variation in outer diameter of the proximal inner tube 230, axial movement of the proximal inner tube 230 relative to the outer delivery tube 215 is restricted when the transition 265 and the stopping member 225 directly physically contact/engage one another. Transition 265 in the exemplary configuration shown in Figure 7 is tapered (sloped) between the proximal and distal sections 245, 250, respectively. Alternatively, a step transition is also contemplated and within the intended scope of the present invention. Other geometric configurations for the transition 265 in outer diameter between the proximal and distal sections of the inner tube are possible. Preferably, the profile of the stopping member 225 and transition 265 complement or mirror one another (e.g., the stopping member 225 is V-shape and the transition 265 is tapered (sloped) at a complementary angle). At all times during the delivery and deployment of the implantable intravascular treatment device in the artery of the body the outer delivery tube 215 remains linear or straight (i.e., unbent) and intact (i.e., not broken or severed) to allow unimpeded axial (longitudinal) pulling in a proximal direction of the proximal end 235 of the proximal inner tube 230 through the lumen 220 of the outer delivery tube 215 until further proximal movement is halted when the transition 265 and stopping member 225 physically contact/engage one another. Direct engagement of the transition 265 with the stopping member 225 in the present inventive design advantageously ensures the proximal inner tube 230 once severed along the severable (weakened) region remains self-contained within and prevented from falling out from the proximal end of the outer delivery tube 215, as described in further detail below.

Still referring to Figure 7, distal section 250 of proximal inner tube 230 comprises a cylindrical tube including two components 250a, 250b (hereinafter referred to as first and second distal section components, respectively) having an axially separable (e.g., axially severable) region 250c disposed therebetween.

The stopping member 225 is located a predetermined distance in a proximal direction from the transition 265 (when the assembled delivery and detachment device is in the delivery state) so that before engaging one another sufficient axial movement in a proximal direction of the first distal section component 250a relative to the stationary second distal section component 250b is permitted to release the implantable intravascular treatment device 280.

**In** the exemplary configuration of Figure 6 axially severable region 250c comprises a plurality of frangible struts or connectors 260 spanning between the respective two distal section components 250a, 250b (i.e., opposite ends of each strut connected to one of the respective two distal section components 250a, 250b). The frangible struts or connectors 260, for example, represent those remaining sections created following a series of cuts (preferably laser cuts) made radially inward through the cylindrical tube of the distal section 250. **In** the Figures black lines denote the frangible struts or connectors 260 while the white areas between adjacent black struts or connectors represent regions where the material of the cylindrical tube of the distal section 250 of the proximal inner tube 230 has been cut or removed (e.g., via laser cuts). These plurality of radially inward cuts through the distal section 250 of the proximal inner tube creates the severable region or interface 250c that is "weakened" relative to the remaining components 250a, 250b comprising distal section 250 on either side thereof that is devoid or absent of cuts. Second distal component 250b is maintained in position within the outer delivery tube 215 via a restricting element 270 (e.g., an adhesive joint, weld joint or O-ring) preventing or minimizing gross movement in axial direction therein. In the case of an O-ring or other mechanical restricting element the component is preferably maintained in its axial position (e.g., sandwiched between projections). Upon application of a sufficient axial force (e.g., pulling in a proximal direction on the proximal end or sliding) on the proximal inner tube 230, the struts or connectors 260 within the axially severable weakened region 250c break/rupture/sever such that the two distal section components 250a, 250b are no longer connected to one another (i.e., break apart). Once the struts or connectors 260 are severed, the first distal section component 250a and proximal section 245 attached thereto or integral therewith together as a unit are axially slidable in a proximal direction until the transition 265 and stopping member 225 directly engage/physically contact one another. The second distal section component 250b (furthest from the proximal section 245) is maintained in place within the lumen 220 of the outer delivery tube 215 by the restricting element 270.

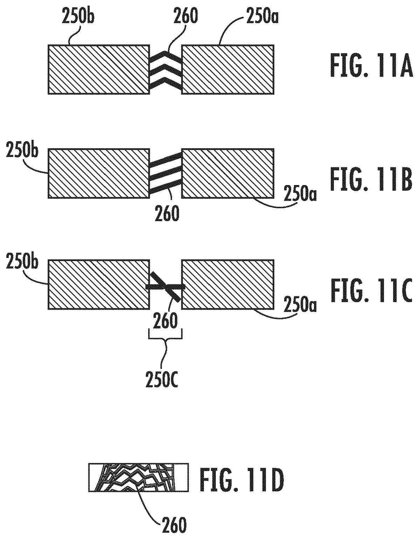

Figure 7 shows an exemplary axially severable region 250c having a plurality of parallel angled connectors or struts 260 formed by a radial spiral cut penetrating radially inward through the proximal inner tube 230 from its outer surface to the channel 255 defined axially therethrough. Other arrangements or patterns (parallel, non-parallel, random, linear, curved, angular, etc.) of the plurality of struts or connectors 260 in the axially severable (weakened) region or interface 250c are contemplated and within the scope of the present invention including, but not limited to, a chevron V-pattern (Figure 11A), an angular pattern relative to the longitudinal axis of the inner tube (Figure 11B), or an intersecting or cross-over (e.g., X-pattern) (Figure 11C). The struts or connectors 260 in the patterns represented in Figures 11A-11C extend/run in an axial direction (longitudinally) spanning between the two distal section components 250a, 250b; however, the struts and connectors 260 disposed between the two distal section components 250a, 250b (as shown in Figure 11D) may be arranged as a radial pattern with offset auxiliary/supplemental struts or connectors extending (running) between adjacent radial rows in an axial (longitudinal) direction. The number (i.e., two or more), spacing between, as well as the dimensions and pattern of the struts or connectors may be modified, as desired, to prevent premature rupture during delivery yet, upon application of a predetermined axial force on the proximal end of the proximal inner tube, ensure reliable and complete deployment/detachment (e.g., breakage, rupture, freeing) of the implantable intravascular treatment device at an intended location within the artery (once properly positioned at the target site).

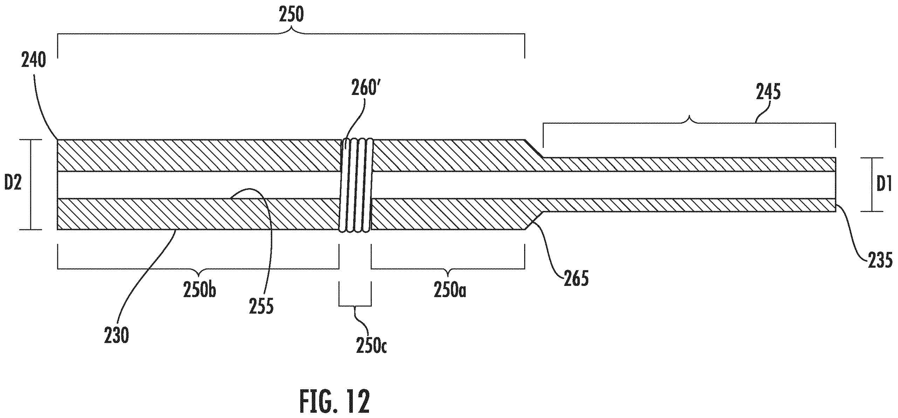

Rather than be severable, in an alternative configuration illustrated in Figure 12 axial section 250c may be expandable in an axial direction (without severing, rupturing or breaking) wherein the two distal section components 250a, 250b remain fixedly connected to respective ends of a helical spring 260' disposed therebetween. When the interventionalist pulls in a proximal direction on the proximal end or slides in a proximal direction the proximal inner tube 230 the helical spring 260' elongates or expands in an axial direction without breaking/rupturing/severing/disconnecting from the respective distal section components 250a, 250b. In all other respects the two embodiments (e.g., axially severable region including frangible struts vs. axially expandable region including an axially expandable helical spring) are the same and the description herein is applicable for both. Other types of axially expandable mechanical devices may be substituted for the helical spring. Rather than a separate component helical spring 260' (as depicted in Figure 12), a helical or spiral cut (e.g., laser cut) may be made to the proximal inner tube 230 along axial section 250C acting in a similar manner.

The implantable intravascular treatment device (e.g., embolic coil) is attached to the delivery and detachment system via a releasable securement mechanism that is released exclusively via mechanical translation (e.g., movement in an axial or longitudinal direction and/or torque) without the need for application of electrical and/or thermal heat.

The present inventive delivery and detachment system is used by the interventionalist to deliver and deposit the implantable intravascular treatment device to the target site in the artery. Maintaining the proper position of the securement wire during delivery of the implantable intravascular treatment device to the target site within the vessel followed thereafter by complete detachment or release of the implantable intravascular treatment device for deposit at the desired target location in the body are instrumental factors in ensuring the success of the endovascular treatment procedure. Undesirable gross shifting, translation, or movement of the securement wire during delivery (prior to deployment/detachment/release) of the implantable intravascular treatment device at the target site may result in damage or injury to the vessel wall. At the time of deployment when the implantable intravascular treatment device is properly located at the target site in the vessel, hampering or total failure in detachment/release of the implantable intravascular treatment device from the wire to which it is secured may result in procedural delay and potentially require withdraw of the implantable intravascular treatment device altogether to be replaced by another. Either potential problem, under certain circumstances, may result in delay, injury to the patient and under some circumstances possibly death. With these goals in mind, the present inventive delivery and detachment system minimizes gross movement of the securement wire during delivery resulting in precise deposit at the target site while allowing fine tuning of the axial force (e.g., pulling) in a proximal direction to reliably release (deploy) the implantable intravascular treatment device. The present inventive integrated and simplified system design provides precise (accuracy of positioning) and safe (minimizing the potential for injury) control during both delivery and detachment/deployment of the implantable intravascular treatment device within the vessel.

Referring to Figure 5, the illustrated exemplary releasable securement mechanism includes a securement wire (e.g., pull wire) 275 extending axially through the detachment and deployment system. A proximal end of the securement wire 275 is fixedly attached to the proximal end 235 of the proximal inner tube 230 via a proximal bead 285 (Figure 6). An opposite distal end of the securement wire 275 is releasably secured to the implantable intravascular treatment device (e.g., embolic coil) 280 via the releasable securement mechanism. Specifically, a loop wire 290 extends in an axial direction through the lumen 220 of the proximal inner tube 230. The loop wire 290 is preferably U-shape having two proximal ends secured to a detachment tube 310 (Figure 4) and a closed distal end (forming a loop) that is threaded through (e.g., bent upwards at an acute angle, preferably perpendicular, relative to the axial direction through the lumen 220) an opening of a proximal key 295 (Figure 5). A sufficient amount of the closed end of the loop wire 290 extends perpendicularly through the opening of the proximal key 295 forming a loop through which the free distal end of the securement wire 275 is axially slidable in a distal direction securing the proximal key 295 to the distal end of the delivery and detachment system. The opposite distal end of the proximal key 295 is received within a cavity at the proximal end of the embolic coil 280 and secured in position by a stretch restraint fiber 300 attached to a distal bead or cap 305.

Operation of the present inventive delivery and detachment system depicted in the figures are representative of various sequential states of operation i.e., delivery, detachment, and deposit of the implantable intravascular treatment device, each of which is described in detail.

Figures 8A & 8B depict the present inventive intravascular delivery and deployment system 200 loaded with the implantable intravascular treatment device (e.g., embolic coil) 280 while in a "delivery state", that is, the state while tracking (delivering) the implantable intravascular treatment device (e.g., embolic coil) to a target site in the vessel/artery. Specifically, the releasable securement mechanism securing the implantable intravascular treatment device (e.g., embolic coil) to the distal end of the delivery and detachment system 200 is illustrated in the cutaway distal section view of Figure 8A. The opposite proximal section of the delivery and detachment system with the proximal inner tube 230 telescopically assembled within the outer delivery tube 215 is shown in the axial cross-sectional view of Figure 8B. While in the "delivery state" this embodiment of the present inventive delivery and deployment system 200 exhibits the following characteristics: (i) the implantable intravascular treatment device (e.g., embolic coil) 280 is mechanically attached, secured or connected via the releasable securement mechanism (e.g., pull wire 275, proximal key 295, and stretch resistant fiber 300) to the distal end of the delivery and detachment system; (ii) the two distal section components 250a, 250b comprising distal section 250 of the proximal inner tube 230 are connected, secured or attached to one another via the severable (weakened) region or interface 250c which is intact and complete (non-ruptured, non-severed); (iii) transition 265 of the proximal inner tube 230 and stopping member 225 in the outer delivery tube 215 are separated from one another in an axial/longitudinal direction by a maximum distance; and (iv) the proximal end 205 of the outer delivery tube 215 and proximal end 235 of proximal inner tube 230 are separated from one another in an axial/longitudinal direction by a minimum distance. Shielded within the channel 255 of the proximal inner tube 230 which, in turn, is stabilized within the lumen 220 of the outer delivery tube 215 via the restricting element 270 (e.g., adhesive joint, weld joint or O-ring), gross movement or translation of the securement wire 275 during delivery of the implantable intravascular treatment device to the target site in the artery is minimized or prevented.

Once the implantable intravascular treatment device (e.g., embolic coil) is located at the target site within the artery, the interventionalist transitions the delivery and detachment system to the next state, hereinafter referred to as a "deployment state" or "detachment state." In order to transition states, the interventionalist subjects the proximal inner tube 230 to movement (e.g., a pulling or sliding force) in a proximal direction relative to that of the outer delivery tube 215.

Deployment begins by manually rupturing (e.g., manually snapping) the struts or connectors 260 in the axially severable (weakened) region 250c upon the application of sufficient axial force on the proximal inner tube 230 in a proximal direction. Specifically, the particular pattern of the struts or connectors 260 formed in the cylindrical inner tube after making the radially inward laser cuts is specifically designed to break, sever, detach or rupture when the proximal inner tube 230 is subject to a predetermined force (e.g., pulling/sliding) in a proximal direction. For this particular design having the axially severable region 250c there is preferably no intended allowance for preliminary stretching in an axial or longitudinal direction of the struts relative to one another before breaking. In other words, in this particular embodiment movement of the proximal inner tube in a proximal direction is for the sole purpose and intent of breaking or severing of the frangible connectors 260.

While in the deployment state, a series of cutaway views of the distal section is shown in Figures 9A-9C during various sequential stages of retraction (movement) in a proximal direction of the proximal inner tube 230. Deployment is accomplished via application of a simplified hand movement exclusively or solely in an axial direction applied to the proximal inner tube making the system user friendly by the interventionalist. Sufficient linear force is applied to rupture or break the frangible struts or connectors 260 within the axially severable (weakened) region or interface 250c permanently severing the distal end 250 into its respective two distal section components 250a, 250b. Specifically, the two distal section components comprise a first distal component 250a including the transition 265 and a second distal section component 250b arranged in a distal direction relative to the first distal section component 250a.

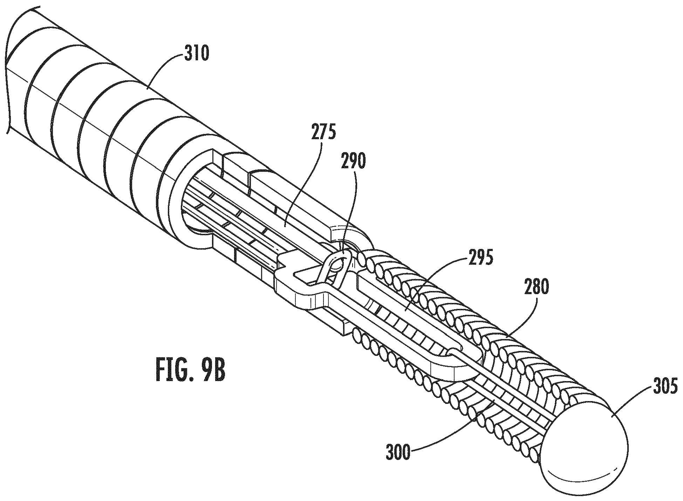

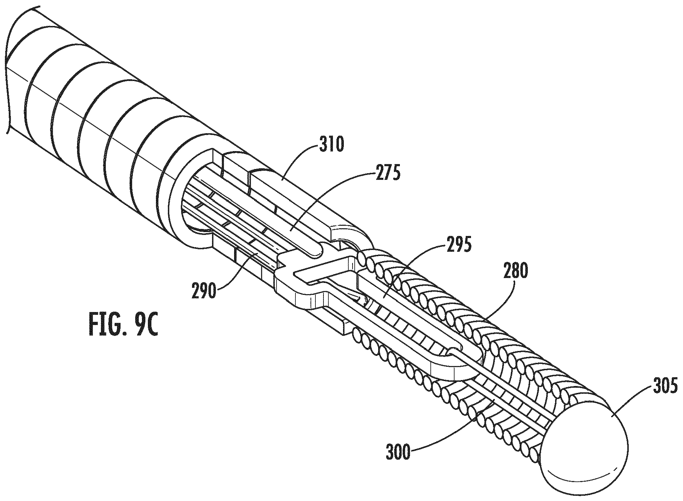

At the time of rupture of the struts or connectors 260, the distal end of the securement wire 275 is retained/secured (e.g., threaded or passed) through the opening at the closed distal end of the U-shape loop wire 290 that is bent upwards through the opening in the proximal key 295, as shown in Figure 9A. Continued retraction (i.e., movement in an axial direction) of the proximal inner tube 230, causes the securement wire 275 connected thereto via the proximal bead 285 to simultaneously therewith move in the same direction (as shown in Figure 9B). At this stage the implantable intravascular treatment device 280 (e.g., embolic coil) is still retained/secured/attached to the distal end of the delivery and detachment system via the bent upwards U-shape loop wire 290 threaded through the opening of the proximal key 295. While still further retraction (i.e., axial movement in a proximal direction) of the proximal inner tube 230 completely withdraws freeing the distal end of the securement wire 275 from the bent upwards U-shape loop wire 290 allowing the U-shape loop wire to substantially straighten (substantially axial or linear) clearing/freeing/releasing it from the proximal key 295 (as shown in Figure 9C).

The opposite distal end of the delivery and detachment system during the deployment state is shown in Figure 9D denoting via the arrows the pulling (i.e., movement in a proximal direction) of the proximal inner tube 230 and the ruptured struts 260 (i.e., axial separation between the first and second distal section components 250a, 250b, respectively). As is also evident from Figure 9D, following rupture of the struts 260, the second severed distal section 250b remains securely in position within the outer delivery tube 215 via the restricting element 270.

During the "deployment state," sufficient axial force in a proximal direction is imparted on the proximal inner tube so that the delivery and detachment system exhibits the following characteristics: (i) the two distal section components 250a, 250b comprising distal section 250 are detached/ruptured/severed/broken apart of one another (i.e., the struts/connectors 260 in the severable (weakened) region or interface 250c are severed, broken, detached from one another); (ii) the releasable securement mechanism is released freeing the implantable intravascular treatment device at its current position within the artery; (iii) transition 265 of the proximal inner tube 230 and stopping member 225 in the outer delivery tube 215 are separated (not engaging/contacting) from one another in an axial/longitudinal direction by a distance less than the maximum distance (while in the "delivery state" of Figure 8B); and (iv) the proximal end 205 of the outer delivery tube 215 and proximal end 235 of the proximal inner tube 230 are separated from one another in an axial/longitudinal direction by a distance greater than the minimum distance (while in the "delivery state" of Figure 8B).

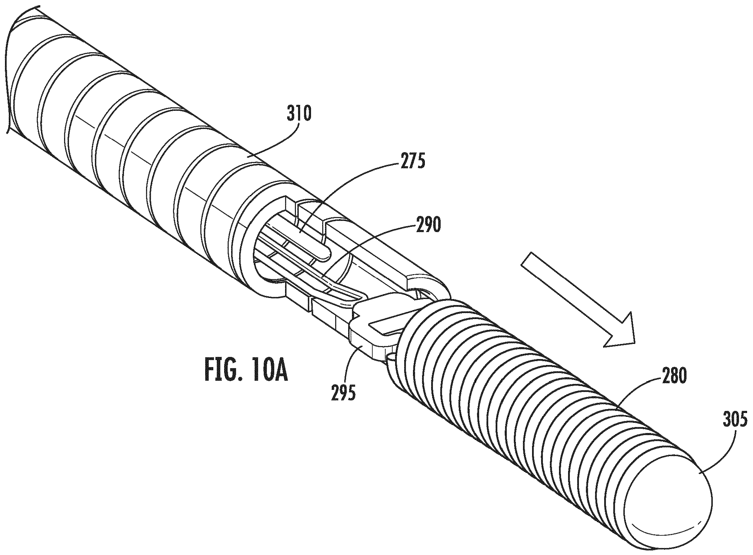

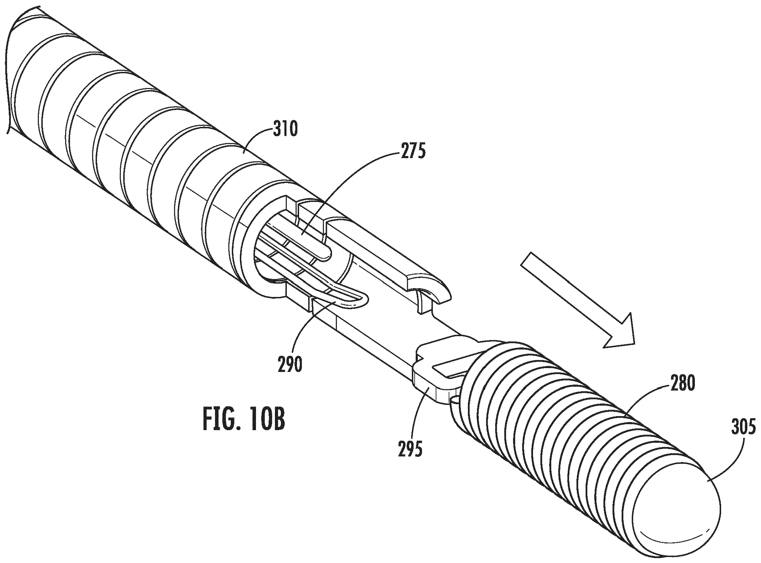

During the transition from the detachment to the deposit state, with the U-shape loop wire 290 no longer secured to (free and clear of) the proximal key 295, a pre-load of the outer delivery tube 215 pushes or advances (as depicted by the arrow in Figures 10A & 10B) in a distal direction the implantable intravascular treatment device (e.g., embolic coil) 280 away from the loop wire 290 facilitating separation of the components from one another. The implantable intravascular treatment device (e.g., embolic coil) 280 together with the proximal key 290 attached thereto via the stretch resistant fiber 300 as a unit remain (e.g., left behind) located/deposited at the target site in the artery.

Referring to the corresponding proximal end shown in Figure 10C, during the depositing stage even further retraction (i.e., axial movement in a proximal direction) of the proximal inner tube 230 maximizes the axial separation of the first distal section component 250a, relative to that of the second distal section component 250b held in position within the outer delivery tube 215 via the restricting element 270. Eventually, retraction of the proximal section 245 together with the severed first distal section component 250a as a unit is limited or restricted by the transition 265 engaging or contacting the stopping member 225 (e.g., crimping or protrusion) (hereinafter referred to as a "halted" state) (Figure 10C). While in the "halted state" (Figure 10C) this embodiment of the present inventive delivery and detachment system exhibits the following characteristics: (i) the two distal section components 250a, 250b are separated from one another in an axial direction a maximum distance (greater than the distance separation during the deployment state of Figure 9D); (ii) the releasable securement mechanism securing the implantable intravascular treatment device (e.g., embolic coil) to the securement wire 275 remains released and free from that of the implantable intravascular treatment device at the location in which it is deposited within the artery (which occurred during deployment); (iii) transition 265 and stopping member 225 contact or engage one another halting further axial movement in a proximal direction of the first distal section component 250a relative to that outer delivery tube 215; and (iv) the proximal end 205 of the outer delivery tube 215 and proximal end 235 of the proximal inner tube 230 are separated from one another in an axial/longitudinal direction by a maximum distance greater than the distance in the deployed state (Figure 9D)). In the halted state in Figure 10C the proximal section 245 and the first distal section component 250a together as a unit are prevented from sliding out of the proximal end of the outer delivery tube 215 by engagement of the stopping member 225 with the transition 265. Thus, despite application of continued pulling force engaging features 225, 265 ensure that the proximal section 245 and first distal section component 250a integral therewith as a unit nevertheless remain self-contained in the outer delivery tube 215. During the medical treatment procedure, self-containment of the proximal section 245 and the first distal section component 250a integral therewith as a unit within the outer delivery tube 215 advantageously reduces the number of components or pieces to be withdrawn from the body to thereafter be disposed. While another benefit of the present inventive self-containment feature eliminates possible unwanted catching of the proximal section 245 and first distal section component 250a as a unit on other pieces or components of medical equipment used in the procedure.

In an alternative embodiment, rupture of the struts 260 in the axially severable weakened section 250c may be realized by subjecting the proximal inner tube to a combination of multiple forces rather than force (movement) exclusively in a linear/axial direction. Specifically, while maintaining the position the outer delivery tube 215 the proximal end 235 of the proximal inner tube 230 may be subject first to torque (rotation) to break the struts 260 followed thereafter by a linear/axial force to deploy or release the implantable intravascular treatment device. In this regard, there is no stored energy (e.g., spring) component to the breaking of the struts action (torque), separation or release of the securement wire occurs only via the linear/axial force (pulling), hence the combination of forces. Prior to applying the linear/axial force, the proximal end 235 of the proximal inner tube 230 together as a unit with the first distal section component 250a integral therewith may be initially subject to torque (rotational) force while the second distal section component 250b of the proximal inner tube 230 is maintained in positioned (e.g., resists rotation) by the restricting element 270. Twisting of the first distal section component 250a while the second distal section component 250b remains in place (e.g., resists rotation because of the restricting element 270) cause the struts or connectors 260 within the axially severable weakened region 250c to rupture/break/sever. Once the struts or connectors 260 have been ruptured/broken/severed/destroyed, thereafter application of a linear/axial force applied in a proximal direction to the proximal section 245 together with the first distal section component 250a advancing together as a single unit through the lumen 220 of the outer delivery tube 215 in a proximal direction. While in yet another alternative embodiment, the proximal end 235 of the proximal inner tube 230 may be subject to a purely torque force along a spiral path (i.e., unscrewing a screw) that allows for a torque (unwinding action) to apply against and rupture the struts 260 while simultaneously following an axial path moving the proximal inner tube in a proximal direction.

Regardless of the force (torque and/or linear/axial), application of an angular force (bending) of the proximal inner tube and/or outer delivery tube either independent of one another or together as a unit at a non-zero angle relative to that of the longitudinal axis extending through the delivery and detachment system 200 is to be avoided potentially hampering or altogether preventing sliding of the proximal inner tube relative to the outer delivery tube and unwanted translation of the securement wire.

The operation of the present inventive delivery and detachment system shown in the figures and described above has been for an exemplary configuration in which the first and second distal section components 250a, 250b are connected via an axially severable weakened region 250c comprising a plurality of frangible struts or connectors 260 that are severable/breakable/rupturable resulting in divisible, separated, independent (no longer connected to one another when the struts are ruptured) distal section components 250a, 250b. As previously noted, in the alternative configuration shown in Figure 12 the two distal section components 250a, 250b are connected one at each end of a linearly expandable or elongated helical spring 260' that never ruptures or severs. During operation (e.g., during the deposit stage) the two distal components are separated axially (when the proximal end of the proximal section 245 is subject to an axial force in a proximal direction but remain connected to one another at all times via the axially expanded/elongated spring helical 260' disposed therebetween.

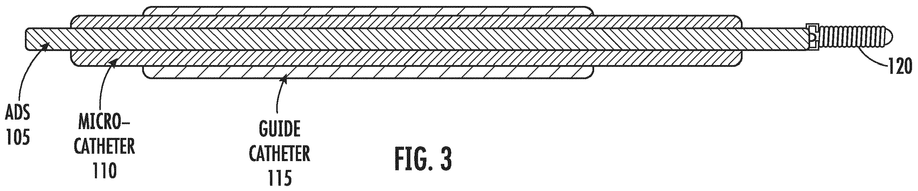

The present inventive delivery and deployment system may be used for implantation of an implantable intravascular treatment device (e.g., embolic coil) in the treatment of an aneurysm. Referring to Figure 3, during one exemplary intravascular treatment procedure, a catheter is navigated through a vessel/or artery until its distal end is positioned on a proximal side of a target site (e.g., aneurysm). Next, a microcatheter is fed through the catheter to the target site. The present inventive delivery and deployment system loaded with the implantable intravascular device (e.g., embolic coil) is together advanced through the lumen of the microcatheter until the implantable intravascular device emerges fully (e.g., clear of or out from) the distal end of the microcatheter. It is noted that the use of a catheter and/or microcatheter advanced to the proximal side of the target site may not be necessary depending on the particular use, treatment or procedure.

## Claims

1. A delivery and detachment system (200) for an implantable intravascular treatment device, the system comprising:
an outer delivery tube (215) with a proximal end (205), an opposite distal end (210), and a lumen (220) extending axially therethrough; the outer delivery tube having a radially inward stopping member (225);
a proximal inner tube (230) telescopically receivable in the distal end and axially slidable in a proximal direction within the lumen of the outer delivery tube; the proximal inner tube having a proximal end, an opposite distal end, and a channel extending axially therethrough; the proximal inner tube comprising a proximal section (245) including the proximal end of the proximal inner tube and a distal section (250) including the distal end of the proximal inner tube; wherein the proximal section and the distal section have different outer diameters forming a transition (265) in outer diameter at an interface therebetween; **characterized in that** the distal section of the proximal inner tube comprises two distal section components connected by an axially separable region (250c); wherein the two distal section components comprise a first distal section component (250a) including the transition and a second distal section component (250b) arranged in a distal direction relative to the first distal section component; and
a restricting element (270) arranged between an outer surface of the second distal section component and an inner wall of the lumen of the outer delivery tube;
when the axially separable region is axially separated: (i) the proximal section of the proximal inner tube is slidable in a proximal direction through the stopping member until the transition in the outer diameter of the first distal section component attached thereto engages the stopping member prohibiting axial movement in the proximal direction; and (ii) the restricting element maintaining in position the second distal section component within the outer delivery tube.

2. The system of claim 1, wherein the radially inward stopping member is a crimping or protrusion; and wherein the restricting element is at least one of an adhesive, a weld, and a mechanical component.

3. The system of claim 1, wherein the axially separable region is an axially severable region having one or more radially inward cuts defined in the proximal inner tube forming a plurality of severable frangible connectors (260).

4. The system of claim 3, wherein the one or more radially inward cuts is a radial spiral cut.

5. The system of claim 3, wherein the plurality of severable frangible connectors extend in an axial direction spanning between the two distal section components; and wherein the one or more radially inward cuts form one of the following patterns:
- a chevron V-pattern;
- an angular pattern relative to a longitudinal axis of the proximal inner tube; and
- an intersecting or cross-over pattern.

6. The system of claim 3, wherein the plurality of severable frangible connectors are arranged as a radial pattern with offset auxiliary/supplemental struts extending between adjacent radial rows in an axial direction.

7. The system of claim 1, wherein the axially separable region is an axially expandable helical spring (260') or a helical cut in the proximal inner tube.

8. The system of claim 1, wherein the transition (265) in outer diameter at the interface between the proximal section and the distal section of the proximal inner tube is a tapered or a step transition.

9. The system of claim 1, wherein when the axially separable region is axially separated both the proximal section together as a unit with the axially separated first distal section component as well as the axially separated second distal section component remain self-contained within the lumen of the outer delivery tube.

## Patentansprüche

1. Abgabe- und Ablösesystem (200) für eine implantierbare intravaskuläre Behandlungsvorrichtung, das System umfassend:
ein äußeres Abgaberohr (215) mit einem proximalen Ende (205), einem entgegengesetzten distalen Ende (210) und einem Lumen (220), das sich axial dahindurch erstreckt; wobei das äußere Abgaberohr ein radial nach innen gerichtetes Stoppelement (225) aufweist;
ein proximales Innenrohr (230), das in dem distalen Ende teleskopartig aufnehmbar und in einer proximalen Richtung innerhalb des Lumens des äußeren Abgaberohrs axial verschiebbar ist; wobei das proximale Innenrohr ein proximales Ende, ein entgegengesetztes distales Ende und einen Kanal aufweist, der sich axial dahindurch erstreckt; das proximale Innenrohr umfassend einen proximalen Abschnitt (245), der das proximale Ende des proximalen Innenrohrs einschließt, und einen distalen Abschnitt (250), der das distale Ende des proximalen Innenrohrs einschließt; wobei der proximale Abschnitt und der distale Abschnitt unterschiedliche Außendurchmesser aufweisen, die an einer Schnittstelle dazwischen einen Übergang (265) in dem Außendurchmesser ausbilden;
**dadurch gekennzeichnet, dass** der distale Abschnitt des proximalen Innenrohrs zwei distale Abschnittskomponenten umfasst, die durch einen axial trennbaren Bereich (250c) verbunden sind; wobei die zwei distalen Abschnittskomponenten eine erste distale Abschnittskomponente (250a), die den Übergang einschließt, und eine zweite distale Abschnittskomponente (250b) umfassen, die in einer distalen Richtung relativ zu der ersten distalen Abschnittskomponente angeordnet ist; und
ein Begrenzungselement (270), das zwischen einer Außenoberfläche der zweiten distalen Abschnittskomponente und einer Innenwand des Lumens des äußeren Abgaberohrs angeordnet ist;
wenn der axial trennbare Bereich axial getrennt ist: (i) der proximale Abschnitt des proximalen Innenrohrs in einer proximalen Richtung durch das Stoppelement verschiebbar ist, bis der Übergang in dem Außendurchmesser der ersten distalen Abschnittskomponente, die daran befestigt ist, in das Stoppelement eingreift, wobei eine axiale Bewegung in der proximalen Richtung verhindert wird; und (ii) das Begrenzungselement die zweite distale Abschnittskomponente innerhalb des äußeren Abgaberohrs in Position hält.

2. System nach Anspruch 1, wobei das radial nach innen gerichtete Stoppelement eine Crimpung oder ein Vorsprung ist; und wobei das Begrenzungselement mindestens eines von einem Klebstoff, einer Schweißnaht und einer mechanischen Komponente ist.

3. System nach Anspruch 1, wobei der axial trennbare Bereich ein axial trennbarer Bereich ist, der einen oder mehrere radial nach innen gerichtete Schnitte aufweist, die in dem proximalen Innenrohr definiert sind, wobei eine Vielzahl von trennbaren, zerbrechlichen Verbindern (260) ausgebildet wird.

4. System nach Anspruch 3, wobei der eine oder die mehreren radial nach innen gerichteten Schnitte ein radialer Spiralschnitt sind.

5. System nach Anspruch 3, wobei sich die Vielzahl von trennbaren, zerbrechlichen Verbindern in einer axialen Richtung erstrecken, die sich zwischen den zwei distalen Abschnittskomponenten erstreckt; und wobei der eine oder die mehreren radial nach innen gerichteten Schnitte eines der folgenden Muster ausbilden:
- ein Chevron-V-Muster;
- ein Winkelmuster relativ zu einer Längsachse des proximalen Innenrohrs; und
- ein sich kreuzendes oder überkreuztes Muster.

6. System nach Anspruch 3, wobei die Vielzahl von trennbaren, zerbrechlichen Verbindern als ein radiales Muster angeordnet sind, wobei sich versetzte Hilfs-/Zusatzstreben zwischen angrenzenden radialen Reihen in axialer Richtung erstrecken.

7. System nach Anspruch 1, wobei der axial trennbare Bereich eine axial ausdehnbare Schraubenfeder (260') oder ein Schraubenschnitt in dem proximalen Innenrohr ist.

8. System nach Anspruch 1, wobei der Übergang (265) in dem Außendurchmesser an der Schnittstelle zwischen dem proximalen Abschnitt und dem distalen Abschnitt des proximalen Innenrohrs ein konischer oder stufenförmiger Übergang ist.

9. System nach Anspruch 1, wobei, wenn der axial trennbare Bereich axial getrennt ist, sowohl der proximale Abschnitt zusammen mit der axial getrennten ersten distalen Abschnittskomponente als Einheit als auch die axial getrennte zweite distale Abschnittskomponente in sich geschlossen innerhalb des Lumens des äußeren Abgaberohrs verbleiben.

## Revendications

1. Système de pose et de détachement (200) pour un dispositif de traitement intravasculaire implantable, le système comprenant :
un tube de pose externe (215) avec une extrémité proximale (205), une extrémité distale opposée (210), et une lumière (220) s'étendant axialement à travers celui-ci ; le tube de pose externe ayant un organe d'arrêt (225) orienté radialement vers l'intérieur ;
un tube interne proximal (230) pouvant être reçu de manière télescopique dans l'extrémité distale et pouvant coulisser de manière axiale dans une direction proximale au sein de la lumière du tube de pose externe ; le tube interne proximal ayant une extrémité proximale, une extrémité distale opposée et un canal s'étendant de manière axiale à travers celui-ci ; le tube interne proximal comprenant une section proximale (245) comportant l'extrémité proximale du tube interne proximal et une section distale (250) comportant l'extrémité distale du tube interne proximal ; dans lequel la section proximale et la section distale ont des diamètres externes différents, formant une transition (265) dans le diamètre externe au niveau d'une interface entre celles-ci ;
**caractérisé en ce que** la section distale du tube interne proximal comprend deux composants de section distale reliés par une région séparable de manière axiale (250c) ; dans lequel les deux composants de section distale comprennent un premier composant de section distale (250a) comportant la transition et un second composant de section distale (250b) disposé dans une direction distale par rapport au premier composant de section distale ; et
un élément de restriction (270) disposé entre une surface externe du second composant de section distale et une paroi interne de la lumière du tube d'administration externe ;
lorsque la région séparable de manière axiale est séparée de manière axiale : (i) la section proximale du tube interne proximal peut coulisser dans une direction proximale à travers l'organe d'arrêt jusqu'à ce que la transition dans le diamètre externe du premier composant de section distale qui y est attaché mette en prise l'organe d'arrêt empêchant un mouvement axial dans la direction proximale ; et (ii) l'élément de restriction maintenant en position le second composant de section distale au sein du tube de pose externe.

2. Système selon la revendication 1, dans lequel l'organe d'arrêt orienté radialement vers l'intérieur est un sertissage ou une saillie ; et dans lequel l'élément de restriction est au moins l'un parmi un adhésif, une soudure et un composant mécanique.

3. Système selon la revendication 1, dans lequel la région séparable de manière axiale est une région divisible de manière axiale ayant une ou plusieurs coupes orientées radialement vers l'intérieur définies dans le tube interne proximal formant une pluralité d'éléments de liaison frangibles divisibles (260).

4. Système selon la revendication 3, dans lequel la ou les coupes orientées radialement vers l'intérieur sont des coupes radiales en spirale.

5. Système selon la revendication 3, dans lequel la pluralité d'éléments de liaison frangibles divisibles s'étend dans une direction axiale entre les deux composants de section distale ; et dans lequel la ou les coupes orientées radialement vers l'intérieur forment l'un des motifs suivants :
- un motif en V à chevrons ;
- un motif angulaire par rapport à un axe longitudinal du tube interne proximal ; et
- un motif d'intersection ou de croisement.

6. Système selon la revendication 3, dans lequel la pluralité d'éléments de liaison frangibles divisibles est disposée selon un motif radial avec des entretoises auxiliaires/supplémentaires décalées s'étendant entre les rangées radiales adjacentes dans une direction axiale.

7. Système selon la revendication 1, dans lequel la région séparable de manière axiale est un ressort hélicoïdal extensible de manière axiale (260') ou une coupe hélicoïdale dans le tube interne proximal.

8. Système selon la revendication 1, dans lequel la transition (265) dans le diamètre externe au niveau de l'interface entre la section proximale et la section distale du tube interne proximal est une transition conique ou en escalier.

9. Système selon la revendication 1, dans lequel, lorsque la région séparable de manière axiale est séparée de manière axiale à la fois la section proximale, ensemble en tant qu'unité avec le premier composant de section distale séparé de manière axiale, et le second composant de section distale séparé de manière axiale, restent autonomes au sein de la lumière du tube de pose externe.
